# EUROPÄISCHE PATENTSCHRIFT

(11) **EP 2 266 660 B1**
(45) Veröffentlichungstag und Bekanntmachung des Hinweises auf die Patenterteilung: **06.06.2012**
(21) Anmeldenummer: 10006234.8
(22) Anmeldetag: 16.06.2010
(51) Int. Cl.: A61N 1/36, A61B 5/117, A61H 39/00

(54) **Elektrostimulationsgerät für mehrere Benutzer**
Electrostimulation device for multiple users
Appareil de stimulation électrique pour plusieurs utilisateurs

(30) Priorität: 27.06.2009 DE 202009008855 U
(43) Veröffentlichungstag der Anmeldung: 29.12.2010
(73) Patentinhaber: Dittmann, Horst, 97727 Fuchsstadt (DE)
(72) Erfinder: Dittmann, Horst, 97727 Fuchsstadt (DE)
(74) Vertreter: Pöhner, Wilfried Anton

(56) Entgegenhaltungen:
- EP-A1- 1 358 842
- WO-A1-99/29367
- WO-A1-2005/009536
- WO-A2-2007/146213

## Beschreibung

Die Erfindung bezieht sich auf ein Elektrostimulationsgerät zur elektrischen Stimulation von Teilen des menschlichen Körpers, bestehend aus einem Gehäuse und einer elektrischen Energiequelle und einer elektronischen Steuerung und wenigstens einem Display und wenigstens einer Bedieneinheit und wenigstens zwei elektrischen Anschlüssen und wenigstens zwei flexiblen Elektrokabeln mit Hautelektroden, wobei an das Elektrostimulationsgerät gleichzeitig zwei oder mehr Nutzer anschließbar sind, wobei vom Gerät individuelle Trainingsprogramme für die Nutzer bei gleichzeitiger Anwendung vorgebbar sind.

Auf aktuellem Stand der Technik ist eine bekannte Variante eines Elektrostimulationsgerätes ein TENS-Gerät zur transkutanen - über die Haut weitergeleiteten - elektrischen Nerven-Stimulation. Die transkutane elektrische Nervenstimulation ist eine elektromedizinische Reizstromtherapie. Sie wird in einer ersten Anwendungsart zur Schmerzlinderung und zur Behandlung von Neuralgien, Durchblutungsstörungen oder orthopädischen Erkrankungen wie Ischalgien, Sehnenscheidenentzündungen, Arthrosen und ähnlichem eingesetzt. Eine zweite Anwendung ist die Stimulation von Muskeln, um sie zu trainieren.

TENS-Geräte geben meist biphasische Stromimpulse mit Frequenzen von etwa 1 - 120 Hz ab. Die schmerzlindernde Wirkung wird mit der "Gate-Control-Theorie", der Kontrollschrankentheorie erklärt. Demnach werden äußere und innere Schmerzreize von Schmerzrezeptoren (sogenannten Nozizeptoren) in Hautmuskeln und Gelenken aufgenommen. Sie werden im Hinterhorn des Rückenmarks auf das zweite Neuron der Schmerzbahn verschaltet. Da hierbei viele Neuronen aus der Peripherie auf ein einziges Neuron konvergieren, wird der Vorgang auch als "Wide-Dynamic-Range-Neuron" (WDR-Neuron) bezeichnet.

Diese Verschaltung unterliegt einer sehr starken Modulation durch andere Neurone: A-Beta-Fasern von sensorischen Afferenzen aus der Peripherie unterdrücken die Weiterleitung. Zu Grunde liegt eine Modulation über Glutamat und metabotrobe Glutamatrezeptoren.

Absteigende, also deszendirende Bahnen hemmen mit einem Transmitter die Übertragung auf das WDR-Neuron oder sie innervieren ein hemmendes Interneuron innerhalb des Rückenmarks. Dieses schüttet dann endogene Opioidpeptide wie Endorphine und ähnliches aus und hemmt über µ-Rezeptoren die Signalweiterleitung auf das WDR-Neuron.

Auf aktuellem Stand der Technik sind die Elektroden auf der Haut aufgeklebt oder durch Sauger oder durch Klemmvorrichtungen befestigt. Durch ein Gel wird der Kontaktwiderstand zwischen Elektrode und Haut verringert.

In einer zweiten Anwendungsart lösen die durch Elektroden verabreichten, elektrischen Impulse Muskelkontraktionen aus. Das Wirkprinzip ist auch allgemein bekannt durch den Herzschrittmacher, der Muskeln des Herzens stimuliert. Die Stärke und die Dauer dieser Impulse kann im TENS-Gerät eingestellt werden. Sie ist Teil des Trainingsprogramms. I.d.R. werden die Impulse nur so schwach dimensioniert, dass sie nicht als unangenehmer Schock empfunden werden und mehrfach pro Sekunde ausgelöst werden.

In dieser Konfiguration dienen die TENS-Geräte zur Erhöhung der Ausdauer des Muskels bei fortwährenden Kontraktionen. Das Ergebnis eines solchen Trainings ist eine Straffung der Muskeln. Die TENS-Geräte sind insbesondere dann sehr hilfreich, wenn ein Nutzer durch andere Erkrankungen, Verletzungen oder Unfälle zur Unbeweglichkeit gezwungen ist. Dann wird ein sich daraus ergebender Abbau der Muskeln durch eine TENS-Therapie zumindest gehemmt oder unterdrückt. Auch bei gesunden Personen kann durch ein regelmäßiges und maßvolles Training die Belastbarkeit und die Ausdauer von Muskeln gesteigert werden, insbesondere in Verbindung mit konventionellem, physischem Training.

Auf aktuellem Stand der Technik bestätig die DE 295 04 054 den Nutzen transkutaner elektrischer Reizstromes zur Aktivierung von Muskeln und deren Aufbau (Seite 2 Absatz 2). Als Nachweis für die klinisch erprobte Wirksamkeit werden die folgenden vier Literaturstellen angeführt.
1. Edel, H. (1977) Fibel der Elektrodiagnostik und Elektrotherapie. Verlag Theodor Steinkopf, Dresden
2. Taylor P.N., Ewins D. J., Fox B., Grundy D., Swain I. D. (1993) Limb blood flow, cardiac output and quadriceps muscle bulk following spinal cord injury and the effect of training for the Odstock functional electrical stimulation standing system. Paraplegia, 31. 303-309
3. Trimble M. H., Enoka R. M. (1991) Mechanisms underlying the training effects associated with neuromuscular electrical stimulation. Phys-Ther, 71: 273-281
4. Delitto A., Snyder-Mackler L. (1990) Two theories of muscle strength augmentation using percutaneous electrical stimulation. Phys-Ther, 70: 158-165

Die DE 295 04 054 stellt zusätzlich zu der bekannten Behandlung der Skelettmuskulatur oder der quergestreiften Muskulatur auch ein Gerät zur Behandlung von glatten Muskelzellen, wie z. B. der Speiseröhre, der Gebärmutter, dem Harnleiter, der Harnblase oder den Schwellkörpern nahe der Harnröhre vor. Die Stimulation der Muskelzellen erfolgt über Oberflächenelektroden. Das Gerät bietet auch die Möglichkeit, intern oder in einer Zusatzeinheit die Behandlungszeiten und die angewendeten Parameter zu speichern und im Nachhinein abzurufen.

Ein wesentlicher Nachteil der hier beschriebenen Gerätschaft ist jedoch, dass die Nutzung nur für jeweils eine einzige Person möglich ist. Wenn ein anderer Nutzer auf das gleiche Gerät zugreift, so muss er selbst oder durch Personal dafür sorgen, dass das passende Trainingsprogramm mit der gebotenen Sicherheit verfügbar gemacht wird und dass die Daten der tatsächlich durchgeführten Behandlung seiner Person korrekt zugeordnet werden.

Ebenfalls ist nicht beschrieben, dass ein Gerät gleichzeitig für mehrere Personen nutzbar ist.

Die WO 2007/146 213 A2 präsentiert ein mit der Hand haltbares, elektronisches Elektrostimulationsgerät, das auch unabhängig vom Netz betrieben werden kann. Über ein Display und Bedienelemente können verschiedene Programme zur Elektrostimulation aktiviert werden. Alternativ werden diese Programme von einem Zentralrechner aus in das Gerät eingegeben. Das Gerät kann mit mehreren Elektrodenpaaren verbunden werden, die in schnellem Wechsel mit elektrischen Impulsen beaufschlagt werden. Dadurch können sogar mehrere Personen gleichzeitig stimuliert werden.

Ein wesentlicher Schwachpunkt ist jedoch, dass das Gerät nicht aktiv überprüft, ob die Stimulationsimpulse auch korrekt den einzelnen Personen zugeordnet werden. Die gesamte Behandlung mehrerer Personen zur gleichen Zeit ist also nur insoweit abgesichert, wie jede Person sich auch tatsächlich die Ihnen zugeordneten Elektroden korrekt anlegt.

Auf diesem Hintergrund hat sich die Erfindung die Aufgabe gestellt, ein TENS-Gerät zu entwickeln, das für mehrere Personen nutzbar ist, die das Gerät gleichzeitig anwenden und sich vom Gerät ihr Trainingsprogramm vorgeben lassen können und die korrekte Durchführung der vorgegebenen Trainingseinheiten speichern und mit korrekter Identität zur späteren Überprüfung wieder abrufen können. Dabei soll das Gerät in einer Ausführungsvariante auch überprüfen, ob die Angabe der Identität korrekt ist.

Als Lösung lehrt die Erfindung, dass die Identität der Nutzer durch das Elektrostimulationsgerät erfassbar und speicherbar ist und vom Gerät individuelle Trainingsprogramme für die Nutzer bei gleichzeitiger Anwendung vorgebbar sind.

In der Grundausstattung eines erfindungsgemäßen Gerätes ist also ein Speicher vorhanden, der zumindest zwei Nutzer voneinander unterscheidet, die ihre Identität dem Gerät mitteilen können. Im einfachsten Fall kann das durch eine Umschaltfunktion geschehen, mit der der Nutzer vorgibt, ob er die Person A oder die Person B ist.

In einer weiteren Ausgestaltung kann auch über eine entsprechende Bedieneinheit ein Name oder ein anderes persönliches Kennzeichen eingegeben werden. Diese Eingabe der Identität schließt ein, dass nicht nur die jeweils verabreichten Impulse mit Spannung, Frequenz, Impulsdauer, Pausenlänge sowie Datum und Uhrzeit des Beginns und des Endes jeder Nutzungsperiode abgespeichert werden, sondern - in einer weiteren Ausführungsvariante - auch dazugehörende Kommandos für den jeweiligen Nutzer abspeicherbar und von ihm abrufbar sind.

Erfindungsgemäße Elektrostimulationsgeräte können für verschiedene Anwendungen in verschiedenen Ausführungen hergestellt werden. Eine Ausführungsvariante ist ein TENS-Gerät zur transkutanen - über die Haut weitergeleiteten - elektrischen Nerven-Stimulation oder Reizstromtherapie. Sie wird in einer ersten Anwendungsart zur Schmerzlinderung und zur Behandlung von Neuralgien, Durchblutungsstörungen oder orthopädischen Erkrankungen wie Ischalgien, Sehnenscheidenentzündungen, Arthrosen und ähnlichem eingesetzt. Eine zweite Anwendung ist die Stimulation von Muskeln, um sie zu trainieren.

Erfindungsgemäße Elektrostimulationsgeräte, die nur der Stimulation von Muskeln dienen sollen, werden auch als EMS-Geräte zur elektrischen Muskelstimulation bezeichnet.

Eine andere Variante ist ein Gerät für die Elektromyographie (EMG). Das ist eine elektrophysiologische Methode der Diagnostik zur Messung der elektrischen Muskel-Aktivität. Durch die Messung von Potenzialänderungen auf der Haut mit Oberflächenelektroden wird das Summen-Aktionspotenzial eines ganzen Muskels oder mehrerer Muskeln erfasst. Durch Vergleich mit Normalwerten kann beurteilt werden, ob das gemessene Potential im Normalbereich liegt oder ob es krankhaft verändert ist. Bei abnormen Werten können myopathische, also in den Muskeln begründete Abweichungen von neuropathischen, also in den Nerven zu suchenden Problemen unterschieden werden.

Eine Kombination der EMG-Funktion mit anderen Ausführungsvarianten eines erfindungsgemäßen Elektrostimulationsgerätes ermöglicht auch ein Biofeedback, also die Messung der körpereigenen Impulse zur Ansteuerung der Muskeln und die Rückkopplung der gemessenen Werte in die Ansteuerung der Stimulationsimpulse.

Im Folgenden soll die Erfindung am Beispiel eines TENS-Gerätes vorgestellt und erläutert werden. Bekanntlich ist ein TENS-Gerät insbesondere dann für den Muskelaufbau und die Muskelverstärkung von ansonsten gesunden und nicht behinderten Personen geeignet, wenn die Nutzung des TENS-Gerätes mit selbst aktivierten Muskelanspannungen kombiniert wird. Hierdurch wird also eine Verstärkung des Trainingseffektes erreicht.

Da jedoch Muskeln empfindlich auf ein Übermaß an Belastung reagieren, ist es höchst sinnvoll, ein regelmäßiges und zuvor geplantes Trainingsprogramm auszuführen, das idealer Weise von einer Fachkraft oder sogar einem medizinisch Geschulten vorgegeben wir. Da Individuen grundsätzlich verschieden sind, wäre es fatal, wenn die Trainingsvorgabe für die eine Person mit den Vorgaben für eine andere Person verwechselt würde oder wenn sogar Trainingsdaten verschiedener Personen miteinander vermischt werden würden.

In einer weiteren, vorteilhaften Ausstattungsvariante sind im TENS-Gerät Messwerte des jeweiligen Nutzers während seiner Nutzungsphasen speicherbar. Sinnvoll sind z. B. die Speicherung der Hauttemperatur und/oder des elektrischen Widerstandes auf der Haut und/oder die zeitliche Änderung der Hauttemperatur oder des elektrischen Widerstandes. Aus einer Tabelle dieser Daten kann der Fachkundige den Erfolg des Trainings unter medizinischen Gesichtspunkten beurteilen, Rückschlüsse für die Vorgabe der weiteren Trainingseinheiten ziehen, wie z. B. eine mögliche Steigerung von Intensität und Anzahl der Nutzungsperioden und der jeweiligen Muskelstimulation innerhalb dieser Perioden und daraus ein differenziertes Trainingsprogramm ableiten. Es beinhaltet genaue Vorgaben für die Anspannung und Entspannung der Muskeln nach Art des Muskels, Anzahl der Anspannungen und Entspannungen sowie über die Zeitdauer der Anspannung und der Entspannung.

Falls der Nutzer die vorgegebenen Zeiten unter - oder überschreitet ist eine Erfassung möglich. Das Gerät kann bei Unterschreitung der vorgegebenen Grenzen zu nachträglichen, weiteren Trainingseinheiten mit entsprechender Verstärkung auffordern. Bei drohendem Überschreiten von vorgegebenen Grenzen oder vorgegebenen Zeiten kann mit einem Warnsignal auf die Beendigung der Trainingsperiode oder des Trainings dieses Muskels hingewiesen werden.

Eine weitere wesentliche Funktion ist eine Terminüberwachung, die an die nächste, fällige Nutzungsperiode erinnert. Das TENS-Gerät und andere, erfindungsgemäße Elektrostimulationsgeräte können mit einer Vorlaufzeit und zum jeweiligen Termin optisch und/oder akustisch an den Termin erinnern. Zusätzlich kann es über eine drahtlose Schnittstelle, z.B. per Blue Tooth, ein Telefongerät aktivieren oder durch Integration der relevanten Teile eines Mobiltelefons einen Telefonanruf auslösen.

Die Einhaltung der korrekten Zeitpunkte ist beim Muskelaufbau insbesondere deshalb sehr wichtig, da der Muskel bekanntlich nicht während des Trainings, sondern erst danach in den Ruhepausen die gewünschte Stärkung aufbauen kann. Deshalb sind ein sinnvolles Maß sowie die Regelmäßigkeit des Trainings für den Muskelaufbau von ganz wesentlicher Bedeutung.

Wenn mehrere Personen ein und dasselbe Gerät gleichzeitig oder nacheinander nutzen sollen, so wäre es, wie bereits mehrfach erwähnt, verhängnisvoll, wenn hierbei Vorgaben und/oder Daten einer Person mit anderen Nutzern verwechselt werden würden. Deshalb ist es eine höchst sinnvolle Ausstattungsvariante eines erfindungsgemäßen Elektrostimulationsgerätes, dass es in einem Verfahren zu Beginn einer jeden Nutzungsperiode überprüft, ob der jeweilige Nutzer sich auch unter der korrekten Identität angemeldet hat.

In einer verfeinerten Variante ist es also denkbar, dass die Identität automatisch erfasst wird und dem Nutzer nur das Ergebnis vorgelegt wird, das er dann notfalls auch korrigieren kann. Dabei wird als eines von mehreren möglichen Verfahren zur Ermittlung der Identität einer Personen vorgeschlagen, dass beim erstmaligen Nutzen des Gerätes durch eine Person in einem normierten Verfahren bestimmte, nur für diese Person zutreffende Daten ermittelt werden und bei jeder weiteren Nutzung zu Beginn der Nutzungsperiode in einem gleichen Zyklus wiederholt abgefragt werden und nur bei Einhaltung der Messwerte innerhalb eines gewissen Bereiches die Übereinstimmung der Identität festgestellt und gemeldet wird.

Dazu wird vorgeschlagen, dass das Gerät vorgibt, die Elektroden an einen bestimmten Muskel anzulegen. Es gibt dann Kommandos zum maximalen Anspannen und zum Entspannen dieses Muskels mit einer jeweils konkreten Zeitvorgabe. Für beide Muskelzustände werden der elektrische Widerstand und/oder dessen Änderung in Abhängigkeit von der Zeit erfasst und beim ersten Nutzen abgespeichert. Zu Beginn der nächsten Nutzungsperiode wird als Bestätigung für die Identität des jeweiligen Nutzers das Anlegen der Elektroden an die gleichen Stellen und das Anspannen und Entspannen des jeweiligen Muskels mit den gleichen Zeiten für jeden Abschnitt vorgegeben.

Falls der gemessene elektrische Widerstand jeweils in einem vorgegebenen Toleranzfeld gleich ist, nennt das Gerät die Identität des jeweiligen Benutzers. Das kann im einfachsten Fall das Aktivieren einer Leuchtdiode für den Benutzer A oder den Benutzer B sein. Alternativ kann mit wachsender Ausstattung des Gerätes der Name, ein Foto oder die Stimme des jeweiligen Benutzers wiedergegeben werden.

Eine weitere, sinnvolle Variante ist, dass am Ende eines jeweiligen Teilschrittes der vorgegebenen Benutzung der Benutzer über die Bedieneinheit, also per Tastendruck oder Knopfdruck oder Druck auf ein Touch-Screen-Panel die korrekte Durchführung des Schrittes bestätigt und damit zugleich den nächsten Schrittes auslöst. Diese Eingabe kann alternativ auch akustisch erfolgen, sodass der Nutzer nicht stets wechselnd eine Hand freimachen muss, sondern sich mechanisch ungestört der Fortsetzung des Trainings widmen kann.

In einer weiteren, sehr interessanten Ausführungsvariante wird zusätzlich der Blutdruck während der Nutzung gemessen und gespeichert. Nach bekanntem Stand der Technik ist es dazu nötig, eine Manschette an einen Körperteil anzulegen und den Druck des dadurch aufgestauten Blutstromes zu messen. Eine solche zusätzliche Einheit ist auf aktuellem Stand der Technik vergleichsweise kostengünstig. Deshalb ist es sinnvoll, sie mit einem erfindungsgemäßen TENS-Gerät zu kombinieren. Dafür ist es möglich, beide Funktionen in ein gemeinsames Gehäuse zu integrieren. Alternativ kann ein Blutdruckgerät auch als separate Baugruppe konfiguriert werden z. B. als ein Handgelenks-Gerät, das lediglich über eine elektrische oder über eine Funkverbindung mit dem Elektrostimulationsgerät kommuniziert.

In ähnlicher Art und Weise kann ein zur Überwachung des körperlichen Trainings sehr wichtiges Elektrokardiogramm (EKG) während des Trainings gemessen und in einem zentralen Elektrostimulationsgerät gespeichert und später wieder abgerufen werden, z. B. beim behandelnden Arzt, der dann nicht auf womöglich vage oder geschönte Aussagen des Patienten angewiesen ist, sondern sich auf unbestechlich gespeicherte Daten stützen kann.

Im Folgenden sollen weitere Einzelheiten und Merkmale der Erfindung anhand eines TENS-Gerätes als Beispiel für ein Elektrostimulationsgerät näher erläutert werden. Dieses soll die Erfindung jedoch nicht einschränken, sondern nur erläutern. Es zeigt in schematischer Darstellung:

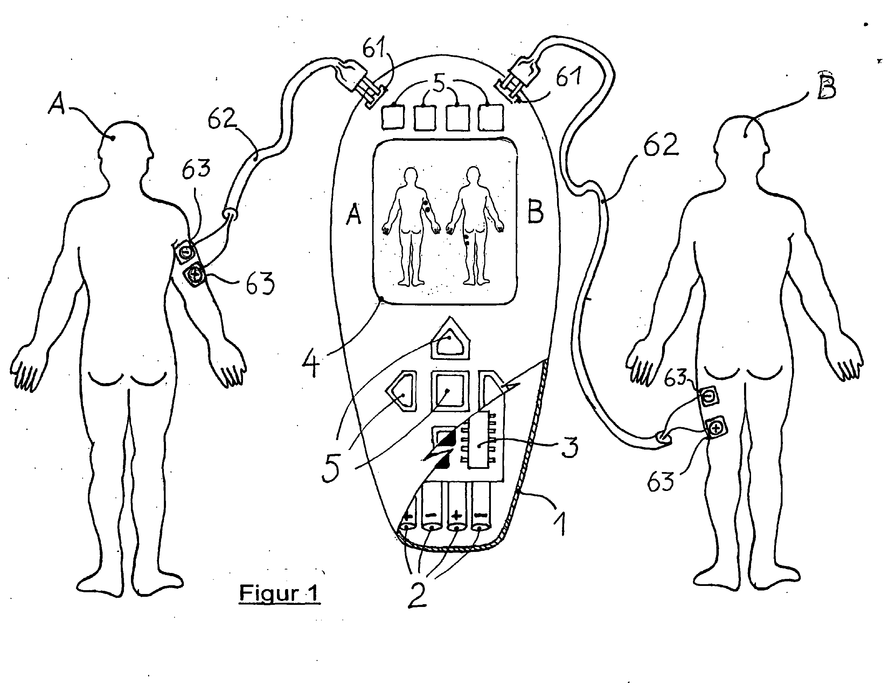

- Figur 1: TENS-Gerät mit zwei Nutzern A und B

In Figur 1 ist die Außenansicht und ein Teil des Inneren eines erfindungsgemäßen TENS-Gerätes mit seinen wesentlichen Merkmalen gezeichnet, an dem ein Nutzer A und ein weiterer Nutzer B angeschlossen sind. Das TENS-Gerät ist in einer Größe gezeichnet, die in etwa einer praktischen Realisierung entspricht und daneben sind zwei im Verhältnis dazu ganz stark verkleinerte Personen als Nutzer A und Nutzer B dargestellt.

In Figur 1 ist das Gehäuse 1 unten rechts zeichnerisch aufgeschnitten um den Blick auf die elektrischen Energiequellen 2 - hier in Form von elektrischen Batterien - freizugeben. Sichtbar wird dabei auch die elektronische Steuerung 3, hier dargestellt durch einen elektronischen Baustein auf einer Platine.

Auf dem Gehäuse 1 ist das Display 4 in der Mitte des Gerätes und mehrere Bedieneinheiten 5 darüber und darunter angeordnet. Auf dem Display 4 sind die beiden Benutzer A und B dargestellt, auf deren silhouettenhaft wiedergegebenem Körperumriss durch schwarze Punkte markiert ist, dass beim Nutzer A der rechte Oberarm elektrisch stimuliert wird und beim Nutzer B der linke Oberschenkel. Dementsprechend sind beim rechts dargestellten Nutzer B am Oberschenkel je zwei Hautelektroden 63 befestigt, die über ein flexibles Elektrokabel 62 mit dem Anschluss 61 für den Nutzer B verbunden sind. In gleicher Weise ist auf der linken Seite des TENS-Gerätes ein Anschluss 61 für ein weiteres, flexibles Elektrokabel 62 angeordnet, das ebenfalls an zwei Hautelektroden 63 angeschlossen ist, die auf dem rechten Oberarm des Nutzers A befestigt sind.

Der in Figur 1 dargestellte Zustand des Gerätes ist für die Inbetriebnahme der Elektroden sinnvoll, bei der der Nutzer selber zu verantworten hat, dass er die Hautelektroden 63 auch an denjenigen Körperbereichen befestigt, die im Display vorgegeben sind. Für diese Körperbereiche wird dann das entsprechende Trainingsprogramm vorgegeben und die Daten des tatsächlich durchgeführten Trainings abgespeichert.

In Figur 1 ist nachvollziehbar, dass nach Anlagen der Körperelektroden an einem vorgegebenen Körperpunkt auch eine Bewegung dieses Körperteiles vorgegeben werden kann, deren charakteristische Daten wie Hautwiderstand und dessen Änderung bei schlagartiger Bewegung abgespeichert werden. Bei einem erneuten Verkabeln des Nutzers A mit dem für ihn vorgesehenen Anschluss 61 kann das erfindungsgemäße TENS-Gerät wiederum abfragen, ob bei der Vorgabe der gleichen Bewegung wie bei der Erstnutzung durch Nutzer A auch die gleichen Messwerte für den Hautwiderstand und dessen Änderung bei schneller Bewegung messbar sind. Wenn ja, quittiert das Gerät diesen Vorgang mit einer Meldung wie z. B. "Benutzer A positiv identifiziert". Dann kann das Gerät auf dem Display die vorgeschriebenen Bewegungskommandos wiedergeben.

Ebenso ist in Figur 1 nachvollziehbar, dass das erfindungsgemäße Gerät auch für die Schmerztherapie einsetzbar ist. Dabei dient die Darstellung des jeweiligen Körperteiles für die korrekte Verdrahtung zum schmerzenden Körperbereich des Nutzers A. Auch in dieser Betriebsart können zwei Nutzer A und B das TENS-Gerät gleichzeitig nutzen. Alternativ verdrahtet ein einziger Nutzer zwei Punkte A und B auf seinem Körper gemäß Vorgabe auf dem Display 4.

Die unten dargestellten Bedienelemente 5 in Form von Pfeiltasten können z. B. für die Verstärkung oder die Abschwächung des Schmerzsignals genutzt werden, die beiden anderen um 90° verdreht angeordneten Pfeiltasten dienen zur Auswahl eines anderen Schmerztherapieprogramms.

Darüber hinaus ist in Figur 1 nachvollziehbar, dass sich die beiden Nutzer A, B gleichzeitig einer Therapie unterziehen können, bei der jeweils gleiche oder verschiedene Körperteile behandelt werden. Weiter macht Figur 1 auch plausibel, dass eine paarweise Therapie denkbar ist, bei der die beiden Personen während der Therapie interaktiv in Kontakt miteinander treten, d.h. sich z. B. die Hände reichen oder sich mit ihren Fußsohlen gegenseitig berühren. Dann kann z. B. das Körperteil des Nutzers A elektrisch stimuliert werden und durch die daraufhin bewirken Impulse, das damit in Berührung befindliche Körperteil der anderen Person B von außen stimuliert werden, sodass vielfältige Effekte erzeugt werden, die ein abwechslungsreiches Trainingsprogramm ergeben und dadurch dessen Wirksamkeit unterstützen und verstärken.

### Bezugszeichenliste

- A: erster Nutzer
- B: zweiter Nutzer
- 1: Gehäuse
- 2: elektrische Energiequelle im Gehäuse 1
- 3: elektronische Steuerung im Gehäuse 1, versorgt durch elektri- sche Energiequelle 2
- 4: Display auf Gehäuse 1
- 5: Bedieneinheiten auf Gehäuse 1
- 61: Anschluss im Gehäuse 1
- 62: Elektrokabel zum Anschluss an Anschluss 1
- 63: Hautelektrode über Elektrokabel 62 mit Anschluss 61 verbun- den

## Patentansprüche

1. Elektrostimulationsgerät zur elektrischen Stimulation von Teilen des menschlichen Körpers, bestehend aus
- einem Gehäuse 1 und
- einer elektrischen Energiequelle 2 und
- einer elektronischen Steuerung 3 und
- wenigstens einem Display 4 und
- wenigstens einer Bedieneinheit 5 und
- wenigstens zwei elektrischen Anschlüssen 61 und
- wenigstens zwei flexiblen Elektrokabeln 62 mit Hautelektroden 63, wobei an das Elektrostimulationsgerät gleichzeitig zwei oder mehr Nutzer A, B anschließbar sind
**dadurch gekennzeichnet, dass**
- die Identität der Nutzer A,B durch das Elektrostimulationsgerät erfassbar und speicherbar ist und
- vom Gerät individuelle Trainingsprogramme für die Nutzer A,B bei gleichzeitiger Anwendung vorgebbar sind.

2. Elektrostimulationsgerät nach dem vorhergehenden Anspruch 1, **dadurch gekennzeichnet, dass** es
- ein TENS-Gerät zur transkutanen - über die Haut weitergeleiteten - elektrischen Nerven- Stimulation oder
- ein EMS-Gerät zur elektrischen Muskelstimulation oder
- eine Kombination aus einem TENS und einem EMS-Gerät und/oder
- ein EMG- Gerät zur Elektromyographie
ist.

3. Elektrostimulationsgerät nach einem der vorhergehenden Ansprüche, **dadurch gekennzeichnet, dass** die Identität des oder der jeweiligen Nutzer(s) A, B über die Bedieneinheit 5 eingebbar ist.

4. Elektrostimulationsgerät nach einem der vorhergehenden Ansprüche, **dadurch gekennzeichnet, dass** in der elektronischen Steuerung 3 in einem Speicher Daten über die jeweilige Nutzung speicherbar, dem jeweiligen Nutzer A, B zuordenbar und zu einem späteren Zeitpunkt aufrufbar und ablesbar und/auslesbar sind.

5. Elektrostimulationsgerät nach einem der vorhergehenden Ansprüche, **dadurch gekennzeichnet, dass** im Speicher
- die Hauttemperatur und/oder
- der elektrische Widerstand und/oder
- deren zeitliche Änderung
speicherbar und abrufbar sind.

6. Elektrostimulationsgerät nach einem der vorhergehenden Ansprüche, **dadurch gekennzeichnet, dass** der korrekte Sitz der Elektroden 63 durch die Messung des Widerstandes zwischen zwei Elektroden 63 und die Auslösung eines Alarmsignals bei Unterschreitung eines Mindestwertes überwachbar ist, wobei das Alarmsignal ein optisches Signal oder ein Sprachsignal oder ein anderes akustisches Signal sein kann.

7. Elektrostimulationsgerät nach einem der vorhergehenden Ansprüche, **dadurch gekennzeichnet, dass** es als TENS-Gerät oder als EMS-Gerät dient und Vorgaben speicherbar und ausgebbar sind für Muskelanspannungen und -entspannungen, beinhaltend
- den jeweiligen Muskel,
- die Anzahl der Anspannungen und/oder
- die Mindestdauer der Anspannung und/oder
- die höchste Dauer der Anspannungen und/oder
- die Mindestdauer der Entspannung und/oder
- die Höchstdauer der Entspannung und/oder
- die Mindestpause und die maximale Pause bis zu nächsten Nutzung und/oder
- eine Alarmfunktion für den Beginn der nächsten Nutzungsperiode und/oder
- akustische oder optische Kommandos für die Muskelanspannung und die Muskelentspannung.

8. Elektrostimulationsgerät nach einem der vorhergehenden Ansprüche, **dadurch gekennzeichnet, dass** es als TENS-Gerät oder EMS-Gerät konfiguriert ist und
- bei der Eingabe der Personenidentität Vorgaben für das Anlegen der Hautelektroden 63 an einem bestimmten Muskel ausgebbar sind und
- Kommandos zum maximalen Anspannen und Entspannen dieses Muskels ausgebbar sind und
- für beide Muskelzustände der elektrische Widerstand und/oder dessen Änderung in Abhängigkeit von der Zeit speicherbar und
- zu Beginn der nächsten Nutzungsperiode als Bestätigung für die korrekte Vorgabe der Identität des jeweiligen Nutzers A, B durch entsprechende Kommandos an den Nutzer A, B überprüfbar sind.

9. Elektrostimulationsgerät nach Anspruch 7, **dadurch gekennzeichnet, dass** am Ende eines jeden Teilschrittes durch den Nutzer A, B über die Bedieneinheit und/oder über ein Mikrophon und eine akustische Eingabe die korrekte Ausführung des jeweiligen Kommandos bestätigbar ist.

10. Elektrostimulationsgerät nach einem der vorhergehenden Ansprüche, **dadurch gekennzeichnet, dass** rechtzeitig vor und bei Erreichung eines Termins des geplanten Trainings- oder Behandlungs-Programms eine Erinnerungsmeldung ausgebbar ist, die
- optisch und/oder
- akustisch und/oder
- drahtlos an ein Telefon
ausgebbar ist.

11. Elektrostimulationsgerät nach einem der vorhergehenden Ansprüche, **dadurch gekennzeichnet, dass** über eine zusätzliche, elektrisch angeschlossene Baugruppe der Blutdruck während der Nutzung messbar und speicherbar ist,

12. Elektrostimulationsgerät nach Anspruch 11, **dadurch gekennzeichnet, dass** ein Blutdruckmessgerät integriert oder angeschlossen ist, bestehend aus einer Manschette, die um den Arm oder das Handgelenk eines Nutzers legbar und aufblasbar ist und einem Messfühler für den Blutdruck während der Nutzung.

13. Elektrostimulationsgerät nach einem der vorhergehenden Ansprüche, **dadurch gekennzeichnet, dass** über zusätzliche Elektroden in der Nähe des Herzens des Nutzers A, B ein EKG (Elektrokardiogramm) aufnehmbar, speicherbar und zu einem späteren Zeitpunkt abrufbar und ausgebbar ist.

14. Elektrostimulationsgerät nach einem der vorhergehenden Ansprüche, **dadurch gekennzeichnet, dass**
- die elektrische Energiequelle 2 wahlweise
- eine nur einmal verwendbare oder
- eine wieder aufladbare Batterie ist und
- in de elektronischen Steuerung 3 die niedrigste Spannung einprogrammierbar ist, bei der noch ein sinnvoller Betrieb möglich ist und
- bei Unterschreiten dieser Spannung eine Alarmmeldung wie z.B. eine LED oder der Schriftzug "battery low" aktiviert wird.

15. Anwendung eines Elektrostimulationsgerätes nach einem der vorhergehenden Ansprüche zum Trainieren von Muskeln nach einem der vorhergehenden Ansprüche, **dadurch gekennzeichnet, dass** die beiden Nutzer A, B je ein zu stimulierendes Körperteil miteinander in Verbindung bringen und eines der beiden, miteinander in Verbindung stehenden Körperteile über darauf angebrachte Hautelektroden 63 stimuliert wird und das andere der beiden, miteinander in Verbindung stehenden Körperteile **dadurch** mit angeregt wird.

## Claims

1. Electrical stimulation device for the electrical stimulation of portions of the human body, comprising
- a housing 1 and
- an electrical energy source 2 and
- an electronic control 3 and
- at least one display 4 and
- at least one operating unit 5 and
- at least two electrical connections 61 and
- at least two flexible electrical cables 62 comprising skin electrodes 63, two or more users A, B being simultaneously connectable to the electrical stimulation device
**characterised in that**
- the electrical stimulation device is capable of registering and storing the identities of users A, B and
- individual training programs for the users A, B can be specified by the device.

2. Electrical stimulation device according to the preceding claim 1, **characterised in that** it is
- a TENS device for transcutaneous- transmitted via the skin - electrical nerve stimulation or
- an EMS device for electrical muscle stimulation or
- a combination of a TENS and an EMS device and/or
- an EMG device for electromyography.

3. Electrical stimulation device according to one of the preceding claims, **characterised in that** the identity of the user or users A, B in each case can be input via the operating unit 5.

4. Electrical stimulation device according to one of the preceding claims, **characterised** that data about the respective use can be stored in the electronic controller 3, in a memory, assigned to the respective user A, B and retrieved and read off and read out at a subsequent time.

5. Electrical stimulation device according to one of the preceding claims, **characterised in that**, in the memory,
- the skin temperature and/or
- the electrical resistance and/or
- the change thereof over time
can be saved and retrieved.

6. Electrical stimulation device according to one of the preceding claims, **characterised in that** the correct seating of the electrodes 63 can be monitored by measuring the resistance between two electrodes 63 and the triggering of an alarm signal when the value falls below a minimum value can be monitored, whereby the alarm signal can be an optical signal or a voice signal or another acoustic signal.

7. Electrical stimulation device according to one of the preceding claims, **characterised in that** it serves as a TENS deice or as an EMS device and settings for muscle tensioning and relaxation can be saved and output, comprising
- the muscle in each case,
- the number of tensionings and/or
- the minimum duration of the tensioning and/or
- the maximum duration of the tensioning and/or
- the minimum duration of the relaxation and/or
- the maximum duration of the relaxation and/or
- the minimum pause and the maximum pause until the next use
and/or
- an alarm function for the start of the next use period and/or
- acoustic or optical commands for the muscle tensioning and the muscle relaxation.

8. Electrical stimulation device according to one of the preceding claims, **characterised in that** it can be configured as a TENS deice or as an EMS device, and
- for inputting the personal identity, settings for application of the skin electrodes 63 to a particular muscle can be output and
- commands for maximum tensioning and relaxation of this muscle can be output and
- for both muscle states, the electrical resistance and/or the change thereof with respect to time can be stored and
- can be examined at the beginning of the next use period by means of corresponding commands to the users A, B, as confirmation of the correct setting of the identity of the user A, B in each case.

9. Electrical stimulation device according to claim 7, **characterised in that**, at the end of each sub-step, the correct execution of the command in each case can be confirmed by the user A, B, by means of the operating unit and/or by means of a microphone and an acoustic input.

10. Electrical stimulation device according to one of the preceding claims, **characterised in that**, promptly before and on reaching a due time for the planned training or treatment programme, a reminder message can be output, which can be output
- optically and/or
- acoustically and/or
- wirelessly to a telephone.

11. Electrical stimulation device according to one of the preceding claims, **characterised in that** the blood pressure can be measured and stored during use via an additional electrically connected module.

12. Electrical stimulation device according to claim 11, **characterised in that** a blood pressure measuring device is integrated or connected, which consists of a sleeve, which can be placed around the arm or wrist and can be inflated, and a measurement sensor for the blood pressure during use.

13. Electrical stimulation device according to one of the preceding claims, **characterised in that**, via additional electrodes in the vicinity of the heart of the user A, B, an ECG (electrocardiogram) can be recorded, stored and retrieved and output at a subsequent time.

14. Electrical stimulation device according to one of the preceding claims, **characterised in that**
- the electrical energy source 2 is optionally
- a battery that can only be used once or
- a rechargeable battery and
- the lowest voltage at which useful operation is still possible can be programmed in the electronic controller 3
- the voltage falls below this value, an alarm message such as an LED or a "battery low" text message is activated.

15. Application of an electrical stimulation device for training of muscles according to one of the preceding claims, **characterised in that** the two users A, B in each case bring a body portion that is to be stimulated into connection and one of the two body portions that are in connection is stimulated by means of skin electrodes 63 applied thereto, and the other of the two body portions that is in connection is co-excited thereby.

## Revendications

1. Appareil de simulation électrique de parties du corps humain, consistant en
• un boîtier 1 et
• une source d'énergie électrique 2 et
• une commande électronique 3 et
• au moins un écran 4 et
• au moins une unité de commande 5 et
• au moins deux raccordements électriques 61 et
• au moins deux câbles électriques 62 avec des électrodes 63, sachant deux ou plusieurs utilisateurs A, B peuvent être raccordés simultanément à l'appareil de simulation électrique
**caractérisé par le fait**
• **que** l'identité des utilisateurs A, B peut être enregistrée et sauvegardée par l'appareil de simulation électrique et
• **que** les programmes individuels de formation peuvent être prescrits pour les utilisateurs A, B pendant une utilisation simultanée

2. Appareil de simulation électrique selon la revendication précédente 1, **caractérisé par le fait**
• **qu'**il s'agit d'un appareil TENS destiné à la simulation électrique transcutanée des nerfs - transmise par la peau - ou
• un appareil EMS destiné à la simulation électrique des muscles ou
• une combinaison d'un appareil TENS et d'un appareil EMS et/ou
• un appareil EMG destiné à l'électromyographie.

3. Appareil de simulation électrique selon une des revendications précédentes, **caractérisé par le fait que** l'identité du ou des utilisateurs respectifs A, B peut être entrée par l'intermédiaire de l'unité de commande.

4. Appareil de simulation électrique selon une des revendications précédentes, **caractérisé par le fait que**, dans la commande électronique 3, des données relatives à l'utilisation respective peuvent être sauvegardées dans une mémoire, peuvent être attribuées à l'utilisateur respectif A, B et être consultées et lues ultérieurement.

5. Appareil de simulation électrique selon une des revendications précédentes, **caractérisé par le fait que**, dans la mémoire,
• la température de la peau et/ou
• la résistance électrique et/ou
• leur changement dans le temps
peuvent être sauvegardés et consultés.

6. Appareil de simulation électrique selon une des revendications précédentes, **caractérisé par le fait que** la bonne fixation des électrodes 63 peut être surveillée à travers la mesure de la résistance entre deux électrodes 63 et le déclenchement d'un signal d'alarme en cas de passage en dessous d'une valeur minimale, sachant que le signal d'alarme est un signal optique ou un signal vocal ou un autre signal acoustique.

7. Appareil de simulation électrique selon une des revendications précédentes, **caractérisé par le fait qu'**il sert en tant qu'appareil TENS ou en tant qu'appareil EMS et que les consignes peuvent être sauvegardées en mémoire et sorties pour les contractions et les décontractions musculaires, comprenant
• le muscle respectif,
• le nombre des contractions et/ou
• la durée minimale de la décontraction et/ou
• la durée maximale des contractions et/ou
• la durée minimale de la décontraction et/ou
• la durée maximale de la décontraction et/ou
• la pause minimale et la pause maximale jusqu'à la prochaine utilisation et/ou
• une fonction d'alarme pour le commencement de la prochaine période d'utilisation et/ou
• les ordres acoustiques et optiques pour la contraction musculaire et la décontraction musculaire.

8. Appareil de simulation électrique selon une des revendications précédentes, **caractérisé par le fait qu'**il est configuré en tant qu'appareil TENS ou qu'appareil EMS et
• que des consignes peuvent être données pour la pose des électrodes cutanés 63 sur un muscle donné et
• que des ordres peuvent être donnés pour la contraction maximale et la décontraction de ce muscle et
• que, pour les deux états du muscle, la résistance électrique et/ou sa modification peuvent être sauvegardée en mémoire en fonction du temps et
• peuvent être vérifiés au début de chaque période d'utilisation en tant que confirmation de l'indication correcte de l'identité de l'utilisateur respectif A, B à travers des ordres correspondants à l'utilisateur A, B.

9. Appareil de simulation électrique selon la revendication 7, **caractérisé par le fait qu'**à la fin de chaque étape partielle, l'utilisateur A, B peut confirmer l'exécution correcte de chaque ordre respectif par l'intermédiaire de l'unité de commande et/ou d'un microphone et d'une entrée acoustique.

10. Appareil de simulation électrique selon une des revendications précédentes, **caractérisé par le fait qu'**un message rappelant le programme d'entraînement ou de traitement peut être émis en temps utile
• sous une forme optique et/ou
• sous une forme acoustique et/ou
• sans fil à un téléphone.

11. Appareil de simulation électrique selon une des revendications précédentes, **caractérisé par le fait que** la tension artérielle peut être mesurée et sauvegardée en mémoire pendant l'utilisation par l'intermédiaire d'un sous-ensemble supplémentaire raccordé électriquement.

12. Appareil de simulation électrique selon une des revendications précédentes, **caractérisé par le fait qu'**un appareil de mesure de la tension artérielle, consistant en une manchette pouvant être mise autour du bras ou du poignet d'un utilisateur et gonflée, et en une sonde de mesure pour la tension artérielle, est intégré ou raccordé.

13. Appareil de simulation électrique selon une des revendications précédentes, **caractérisé par le fait qu'**un électrocardiogramme peut être enregistré, sauvegardé en mémoire, et consulté et sorti ultérieurement par l'intermédiaire d'électrodes supplémentaires à proximité du coeur de l'utilisateur A, B.

14. Appareil de simulation électrique selon une des revendications précédentes, **caractérisé par le fait que**
• la source d'énergie électrique 2 est, au choix,
• une pile utilisable seulement une fois ou
• une pile rechargeable,
• la tension la plus basse avec laquelle un fonctionnement encore sensé est possible pouvant être programmée dans la commande électronique 3,
• un message d'alarme comme par exemple une DEL ou l'inscription « battery low (pile faible) » étant activé au cas où la tension passe en dessous de la valeur programmée.

15. Utilisation d'un appareil de simulation électrique destiné à entraîner des muscles selon une des revendications précédentes, **caractérisé par le fait que** les deux utilisateurs A, B relient chacun une partie de leur corps devant être stimulée et qu'une des deux parties du corps reliée l'une à l'autre est stimulée par des électrodes cutanés 63 posées sur elle, l'autre des deux parties du corps étant de ce fait simultanément stimulée.
